# EUROPEAN PATENT APPLICATION

(11) **EP 4 825 833 A1**
(43) Date of publication of application: **21.10.2026**
(21) Application number: 26171630.2
(22) Date of filing: 10.04.2026
(51) Int. Cl.: C05F 3/06, B09B 3/40, C05F 17/60

(54) **APPARATUS AND METHOD FOR HYGIENISING BIOMASS**

(30) Priority: 17.04.2025 FI 20253141
(71) Applicant: Manpas Oy, 50300 Mikkeli (FI)
(72) Inventor: PÖLÖNEN, Ilpo, 01800 Klaukkala (FI); KUKKAMO, Vesa, 50300 Mikkeli (FI); NISKANEN, Tapani, 90230 Oulu (FI)
(74) Representative: Berggren Oy

(57) **Abstract**

An apparatus for hygienising biomass, such as dry manure or bedded manure, comprises a tank for receiving the biomass to be hygienised, at least one conveyor for transferring the biomass out of the tank, an inner shell which surrounds at least a part of said conveyor, heating means for heating the biomass on the conveyor and a control unit for controlling the operation of the conveyor. The heating means are configured to raise the temperature of said conveyor or a part thereof. Near the initial end of the conveyor there is a first temperature sensor connected to the control unit for measuring the temperature of the biomass on the conveyor, the measuring element of which first temperature sensor is at a distance from the outer surface of the shaft and the inner surface of the inner shell.

## Description

### Field of the invention

The invention relates to is an apparatus and a method for hygienising biomass.

### Prior art

On domestic animal farms, a large amount of manure is generated, which contains animal excrement, bedding materials, urine and food residues. A liquid fraction can be separated from the manure as slurry manure, which can be used as a fertilizer in crop cultivation. The solid fraction of the manure, so-called dry manure, can be further processed so that it can be used as a soil conditioner. The liquid present in the manure can also be absorbed into bedding, such as peat or sawdust, whereby so-called bedded manure is formed, which, when further processed, can be used as a soil conditioner or as bedding.

Untreated dry or bedded manure contains bacteria and pathogens harmful to humans and animals, so the dry or bedded manure must be hygienised before it can be used as litter or as a soil conditioner. Hygienisation can be carried out, for example, by composting. Composting is, however, a slow hygienisation method, since the destruction of pathogens and harmful microbes present in the dry or bedded manure requires a composting time of several weeks. Even a long composting time does not reliably ensure that the dry or bedded manure is completely hygienic after composting.

In agriculture and landscape management, a lot of other biomass-based side streams are also generated, which, when suitably further processed, can be used as soil conditioners or growing media. The use of these side streams also requires that the material is hygienised so that harmful substances possibly contained in the biomass, such as pathogens and existing weed seeds, are destroyed.

The most reliable hygienisation method is to raise the temperature of the biomass to above +70°C for at least one hour, whereby the multiplication of bacteria ceases and the weed seeds lose their germination capacity. Utility model document FI 11655 describes a dry manure hygienisation apparatus, the operating principle of which is based precisely on raising the temperature of the dry manure to said high temperature by means of heating devices present in the apparatus. A weakness of this solution, which functions as such, is that there is no complete certainty about the actual temperature of the dry manure during the heating phase. This may lead to incomplete hygienisation or unnecessarily high energy consumption caused by excessive heating.

The purpose of the invention is to provide hygienisation apparatus and hygienisation method for biomass, such as dry or bedded manure, by which the above-mentioned drawbacks can be reduced. The apparatus and method according to the invention are characterised by what is presented in the independent claims. Some advantageous embodiments of the invention are presented in the dependent claims.

### Brief summary of the invention

The apparatus according to the invention for hygienising biomass, such as dry manure, comprises a tank for receiving the biomass to be hygienised, at least one conveyor for transferring the biomass out of the tank, which conveyor comprises a conveyor screw having a shaft, in which shaft there is a first end and a second end, an inner shell which surrounds at least a part of said conveyor, heating means for heating the biomass on the conveyor and a control unit for controlling the operation of the conveyor. Said heating means are arranged at least partially in said shaft, whereby heat is transferred by conduction from the surface of the shaft to the biomass in direct contact with the shaft. Near the initial end of the conveyor there is a first temperature sensor connected to the control unit for measuring the temperature of the biomass on the conveyor, the measuring element of which first temperature sensor is at a distance from the outer surface of the shaft and the inner surface of the inner shell.

In this description, hygienisation means the inactivation of pathogens and other undesired biological organisms, including weed seeds, by sufficient heat treatment.

In some embodiments of the apparatus according to the invention, the measuring element of said temperature sensor is substantially halfway between the outer surface of the shaft and the inner surface of the inner shell. The term "substantially halfway" means here that the measuring element is located closer to halfway between the outer surface of the shaft and the inner surface of the inner shell than to the outer surface of the shaft or the inner surface of the inner shell. By the placement of the measuring element of the temperature sensor it is ensured that the measurement is carried out from the "middle" of the dry manure at a distance from the hot surfaces of the conveyor. The measurement result thus represents the temperature of the coolest part of the soil biomass conveyed on the conveyor.

In some embodiments of the apparatus according to the invention, said heating means extend from the first end of the shaft to the second end of the shaft, whereby the heating means ensure sufficient heating over the entire length of the conveyor.

In some embodiments of the apparatus according to the invention, there is at least one screw-thread-shaped blade around the shaft of the conveyor, which is attached by its first edge to the shaft and whose free second edge extends close to the inner surface of the inner shell. Heat is transferred by conduction from the shaft to said at least one blade, whereby the surface area of the conveyor in direct contact with the soil conditioner increases and the transfer of heat from the conveyor to the soil conditioner is further enhanced. Preferably, the shaft and the blade are made of a material with good thermal conductivity, such as metal, most preferably steel.

In some embodiments of the apparatus according to the invention, the outer diameter of the first end of said shaft is larger than the outer diameter of the second end of the shaft and the distance of the second edge of said blade from the outer surface of the shaft is smaller at the first end of the shaft than at the second end of the shaft. In these embodiments of the invention, the area delimited by the blade and the outer surface of the shaft at the first end of the conveyor decreases, which further enhances the transfer of heat from the shaft and the blades to the soil conditioner. This accelerates the achievement, at the initial end of the conveyor, of the target temperature set for the heat treatment of the biomass, i.e. the hygienisation temperature.

In some embodiments of the apparatus according to the invention, said control unit is configured to operate the conveyor intermittently so that the conveyor is operated when the temperature of the biomass is higher than the set target temperature and the conveyor is stopped when the temperature of the biomass is lower than the set target temperature.

In some embodiments of the apparatus according to the invention, the first temperature sensor is attached to the inner surface of the inner shell and the second edge of said blade has a recess at the location of said first temperature sensor. Thus, the blade of the rotating conveyor screw does not break the first temperature sensor.

In some embodiments of the apparatus according to the invention, said shaft has a tubular cavity for the flow-through of a heat transfer fluid, which cavity has a first end and a second end, and said heating means comprise a heating boiler for heating the heat transfer fluid, which heating boiler is connected by a supply pipe to the first end of the cavity and by a return pipe to the second end of the cavity, and a pump for circulating the heat transfer fluid from the heating boiler into the cavity and back to the heating boiler. Liquid-circulating heating means are fire-safe. Preferably, the first end of the cavity is at the first end of the shaft and the second end of the cavity is at the second end of the shaft, i.e. the cavity extends over the entire length of the shaft.

Some embodiments of the apparatus according to the invention comprise a second temperature sensor connected to the control unit for measuring the temperature of the heat transfer fluid returning from the shaft to the heating boiler. By monitoring the temperature of the returning heat transfer fluid it can be ensured that the temperature is at the set target level over the entire length of the conveyor, in particular also at the end of the conveyor. If necessary, the heating power can easily be adjusted by increasing the rotational speed of the pump circulating the heat transfer fluid and/or by raising the temperature of the heat transfer fluid.

In some embodiments of the apparatus according to the invention, said heating means additionally comprise a flow channel at least partially around the inner shell, which is connected directly or indirectly to said heating boiler. By circulating the heat transfer fluid also around the inner shell, it can be ensured that the temperature of the biomass remains at the target level over the entire length of the conveyor.

The flow channel can be connected at its first end to the supply pipe and at its second end to the return pipe. In this case, only one pump is needed in the apparatus for circulating the heat transfer fluid and one temperature sensor for measuring the temperature of the heat transfer fluid. Alternatively, the apparatus may have its own pump for circulating the heat transfer fluid in the flow channel and its own temperature sensor for measuring the temperature of the heat transfer fluid returning from the flow channel.

In the method according to the invention for hygienising biomass, such as dry manure, a target temperature is set for the biomass to be hygienised, biomass to be hygienised is received into a tank, biomass is transferred out of the tank by a conveyor having an initial end, an outlet end and an inner shell, and the biomass on the conveyor is heated by heating means to the set target temperature or above at least partially by heating means inside the inner shell. In the method, the temperature of the biomass to be hygienised is measured at the initial end of the conveyor by a first temperature sensor, the measuring element of which is extended into the biomass at a distance from the hottest surfaces of the conveyor.

In some embodiments of the method according to the invention, biomass is transferred by the conveyor towards the outlet end only when the measured temperature exceeds the set target temperature. In this way it can be ensured that the biomass remains above the set target temperature for a sufficiently long time.

In some embodiments of the method according to the invention, a processing time is set for the biomass to be hygienised and the temperature of the biomass on the conveyor is maintained at the target temperature or above for at least said processing time.

In some embodiments of the method according to the invention, dry manure, bedded manure, biowaste, agricultural side stream material or naturally grown reed or straw material is hygienised by the method. Hygienised biomaterial can be used for several different purposes. For example, hygienised bedded manure can be used as litter on animal farms or as a soil conditioner. Hygienised reed and straw material is particularly suitable as a growing medium either as such or mixed with other materials.

An advantage of the invention is that it can be ensured that the biomass to be hygienised remains at the set target temperature for a sufficiently long time for the biomass to be reliably hygienised. For example, bacteria and pathogens contained in dry or bedded manure die at a temperature of +70°C in about one hour. In the same time and at the same temperature, most weed seeds contained in hay, straw and reed materials lose their germination capacity.

A further advantage of the invention is that it is energy-efficient, because heat is transferred efficiently into the biomass and, thanks to accurate temperature measurement, the treatment time of the dry or bedded manure does not need to be extended "just in case".

A particular advantage of one embodiment of the invention is that, thanks to the liquid-circulating heating means, the apparatus is fire-safe, because the soil conditioner cannot overheat to its ignition temperature.

### Brief description of the drawings

In the following, the invention is described in detail. In the description, reference is made to the accompanying drawings, in which
Figure 1 schematically shows an example of an apparatus according to the invention for hygienising biomass as seen from the side, and
Figure 2 schematically shows, as a cross-sectional view, the conveyor part of the apparatus shown in Figure 1.

### Detailed description of the invention

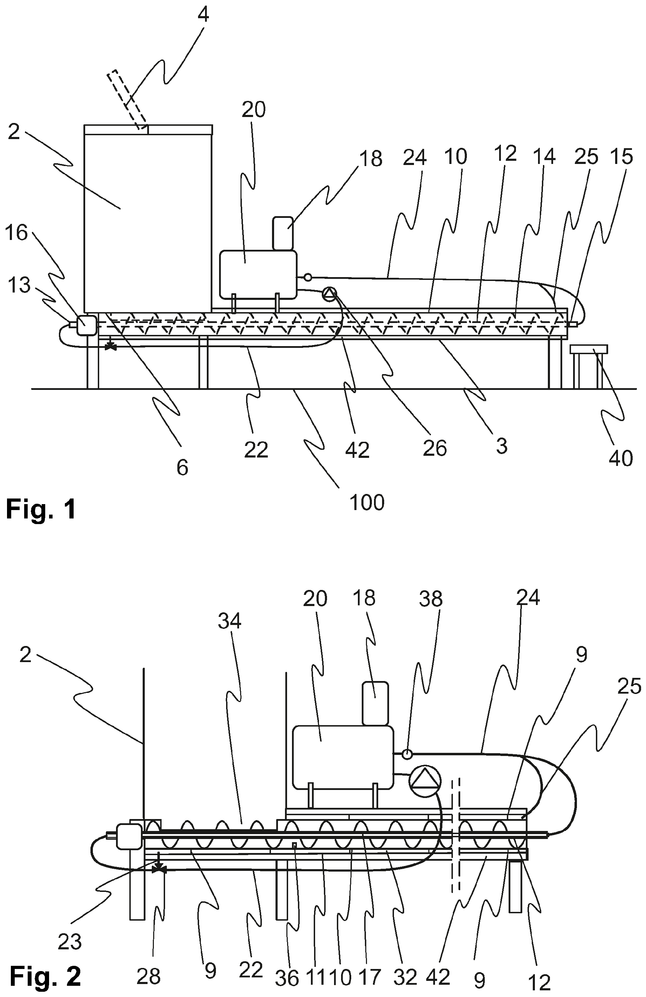

Figure 1 schematically shows, as an example, an apparatus according to the invention for hygienising biomass as seen from the side. In the embodiment of the apparatus shown in Figure 1, the biomass to be hygienised is dry manure, but it could also be bedded manure or some other biomass, such as reed or straw material, either as such or mixed with some additional material.

The apparatus comprises a tank 2, into which the dry manure to be hygienised is dosed through an openable lid 4 at the upper end of the tank. The dry manure may be cattle manure or preferably horse manure from which excess liquid has been removed by means of a separator. The tank functions as a composting space in which the composting of the dry manure starts. The tank has a thermally insulated wall which limits the removal of heat generated in the composting process from the tank and thus promotes the rise of temperature inside the tank. The bottom 6 and the lid of the tank are also preferably thermally insulated. Inside the tank there may be various mixing means for the dry manure and on the inner surface of the tank wall there may be structures preventing excessive arching or compaction of the dry manure.

The size of the tank can be selected. Preferably, the volume of the inner part of the tank is 2-5 m³, more preferably 3-4 m³. The tank may be cylindrical or box-shaped. In a box-shaped tank, instead of an openable lid there may be an openable end wall through which manure is transferred into the tank, and there may be a drag chain conveyor at the bottom of the tank for transferring manure inside the tank.

There is a hole in the bottom of the tank through which the dry manure in the tank can be led to a conveyor 3 located below the tank. The conveyor shown in Figure 1 is a so-called screw conveyor, which comprises a conveyor screw inside a tubular inner shell 10. Around the inner shell there is an outer shell 11 (Figure 2) and a layer of thermal insulation material 42, which may be mineral wool or polyurethane. The conveyor screw has a shaft 12 around which there is a screw-thread-shaped blade 14, which is attached by its first edge to the shaft and whose second edge extends very close to the inner surface of the inner shell. Preferably, there is only a small clearance between the second edge of the blade and the inner surface of the inner shell to allow the conveyor screw to rotate. The shaft and the blade are made of metal, preferably steel, and the blade is attached to the shaft by welding. At the first end of the shaft there is a motor 16 for rotating the shaft. Preferably, the motor is an electric motor. The operation of the motor is controlled by a control unit 18 belonging to the apparatus.

In the portion of the inner and outer shells at the initial end of the conveyor 3 there is an opening 34 (Figure 2), which is aligned with the hole in the bottom 6 of the tank so that the dry manure can flow by gravity from the tank into the spaces between the turns of the blade of the conveyor screw. When the conveyor is operated, i.e. when the shaft of the conveyor screw is rotated by the motor 16, the dry manure is forced by the blades towards the outlet end of the conveyor. The dry manure exits the conveyor through the open second end of the inner shell. The tank and the conveyor are supported by support legs on a load-bearing base 100, for example a floor or yard area.

Below the outlet end of the conveyor there may be a transfer conveyor 40, by which the hygienised dry manure exiting the conveyor is transferred to an intermediate storage to await delivery to the place of use. Preferably, the transfer conveyor is a belt conveyor which moves during the reception of the dry manure, whereby the dry manure coming from the conveyor is spread onto the belt of the transfer conveyor. The inlet temperature of the hygienised dry manure spread onto the transfer conveyor may be 80°C, whereby excess liquid evaporates efficiently from it. By means of guides, the dry manure can be spread onto the transfer conveyor in a layer of suitable thickness, even in a layer less than 1 cm, which increases the evaporation surface area. If necessary, evaporation can be enhanced by infrared heaters, not shown in the figures, which preferably operate intermittently simultaneously with the conveyor and the transfer conveyor.

In the middle of the shaft there is a cavity 17 extending from the first end 13 of the shaft to the second end 15 of the shaft (Figure 2) for the flow-through of a heat transfer fluid. The apparatus further comprises a heating boiler 20, which is connected by a supply pipe 22 to the first end of the cavity and by a return pipe 24 to the second end of the cavity. In the supply pipe there is a pump 26 by which the heat transfer fluid is circulated from the heating boiler through the supply pipe into the shaft and through the return pipe back to the heating boiler. The operation of the pump is controlled by the control unit 18. Alternatively, the rotational speed of the pump can be adjusted manually by an adjustment knob on the pump, in which case the pump does not need to be connected to the control unit.

Figure 2 shows, as a cross-section, a part of the apparatus shown in Figure 1. Inside the inner shell 10 of the conveyor 3, near the opening 34 in the inner shell, there is a first temperature sensor 36, the measuring element of which extends into the space between the blades of the conveyor, at a distance from the hottest surfaces of the conveyor, i.e. from the inner surface of the inner shell and the outer surface of the shaft. Preferably, the location of the measuring element in the radial direction of the shaft is approximately halfway between the inner surface of the inner shell 10 and the outer surface of the shaft 12. The location of the measuring element ensures that the measuring element is positioned inside the dry manure conveyed on the conveyor, whereby the measurement result it provides for the temperature of the dry manure corresponds as closely as possible to the actual temperature of the dry manure.

Around the inner shell 10 surrounding the conveyor screw there is an outer shell 11 such that a substantially uniform-width flow channel 32 for the heat transfer fluid, delimited by the inner and outer shells, remains around the inner shell. On the outer surface of the inner shell, at suitable intervals, preferably at intervals of 0.7-1.3 metres, there are flanges 9 encircling the inner shell, which are attached by their first edge to the outer surface of the inner shell and extend by their second edge to the inner surface of the outer shell. The flanges divide the flow channel into successive compartments in the longitudinal direction of the conveyor. The height of the flanges in the radial direction of the inner shell may be 15-25 mm, preferably about 20 mm. The flanges have through holes which allow the heat transfer fluid to flow through the flanges from one compartment to another. The placement of the holes can be used to influence the flow rate of the heat transfer fluid in different parts of the cross-section of the flow channel. Preferably, the holes are denser and/or the holes have a larger diameter in those portions of the flanges which, in the apparatus in the operating position, are below the lower half of the inner shell. Thus, the flow rate of the heat transfer fluid is higher below the lower half of the inner shell, which can compensate for the effect of the upward movement of the warm heat transfer fluid. In the supply pipe 22 leaving the heating boiler there is a valve 28, preferably a three-way valve, from which a first connecting pipe 23 branches off and is connected to the first end of the flow channel. The second end of the flow channel is connected by a second connecting pipe 25 to the return pipe 24. By means of the valve, a part of the heat transfer fluid coming through the supply pipe can be directed to flow into the flow channel around the shell to heat the inner shell. In the return pipe there is a second temperature sensor 38 connected to the control unit for measuring the temperature of the heat transfer fluid returning to the heating boiler.

The hygienisation of dry manure with the apparatus according to the invention is based on keeping the dry manure to be hygienised at least for a predetermined processing time at a target temperature which is sufficient for the destruction of pathogens and harmful microbes. The target temperature is set in the control unit of the apparatus and its realisation is continuously monitored by measurements. Preferably, the target temperature is at least said 70°C, but it may be higher than this. The conveyor of the apparatus is operated under the control of the control unit so that the dry manure remains inside the inner shell of the conveyor at the target temperature or above for at least the predetermined processing time. In practice, this is best achieved by operating the conveyor in short periods and stopping the conveyor between the periods. A processing time of 1 hour at a temperature of 70°C is sufficient for hygienisation.

The apparatus according to the invention is used in the following way: A suitable amount of dry manure to be hygienised is dosed into the tank and the lid 4 is closed. An aerobic fermentation process starts in the dry manure inside the tank, as a result of which the temperature of the dry manure rises above the target temperature. The heat transfer fluid heated in the heating boiler of the apparatus is circulated by the pump through the shaft of the conveyor and the heat transfer piping, whereby the temperature of the shaft, the blade and the inner shell rises above the target temperature. The shaft of the conveyor is rotated by the motor, whereby the dry manure to be hygienised is transferred towards the outlet end of the conveyor. The first temperature sensor continuously measures the temperature of the dry manure to be hygienised in the conveyor. If the measured temperature falls below the set target temperature, the control unit stops the conveyor. When the conveyor is stopped, heat energy is transferred into the dry manure from the shaft, from the blade heated by the shaft and from the inner shell, whereby the temperature of the dry manure rises. When the measured temperature rises to the target temperature, the control unit starts the motor, whereby the dry manure is transferred towards the end of the conveyor. The control unit operates the conveyor intermittently and continuously measures the temperature of the dry manure at the initial end of the conveyor.

The second temperature sensor continuously measures the temperature of the heat transfer fluid returning to the heating boiler, from which the temperature inside the inner shell of the conveyor can be determined. If the temperature of the returning heat transfer fluid is lower than the target temperature, the control unit increases the rotational speed of the pump pumping the heat transfer fluid and/or the temperature of the heat transfer fluid. The control unit is programmed to operate the conveyor intermittently so that all the dry manure to be hygienised remains inside the conveyor for at least the set processing time. Preferably, the set processing time is one hour, but it may be some other time. This ensures that the dry manure exiting from the second end of the conveyor does not contain live pathogens or harmful microbes.

Above, some advantageous embodiments of the apparatus and method according to the invention have been described. The invention is not limited to the solutions described above, but the inventive idea can be applied in various ways within the limits set by the claims.

### List of reference numerals:

- 2: tank
- 3: conveyor
- 4: lid
- 6: bottom
- 9: flange
- 10: inner shell
- 11: outer shell
- 12: shaft
- 13: first end
- 14: blade
- 15: second end
- 16: motor
- 17: cavity
- 18: control unit
- 20: heating boiler
- 22: supply pipe
- 23: first connecting pipe
- 24: return pipe
- 25: second connecting pipe
- 26: pump
- 28: valve
- 32: flow channel
- 34: opening
- 36: first temperature sensor
- 38: second temperature sensor
- 40: transfer conveyor
- 42: thermal insulation material
- 100: base

## Claims

1. Apparatus for hygienising biomass, such as dry or bedded manure, which apparatus comprises a tank (2) for receiving the biomass to be hygienised, at least one conveyor (3) for transferring the biomass out of the tank (2), which conveyor (3) comprises a conveyor screw having a shaft (12), in which shaft (12) there is a first end (13) and a second end (15), an inner shell (10) which surrounds at least a part of said conveyor (3), heating means for heating the biomass on the conveyor (3) and a control unit (18) for controlling the operation of the conveyor, **characterised in that** said heating means are arranged at least partially in said shaft (12) and that near the initial end of the conveyor (3) there is a first temperature sensor (36) connected to the control unit (18) for measuring the temperature of the biomass on the conveyor (3), the measuring element of which first temperature sensor (36) is at a distance from the outer surface of the shaft (12) and the inner surface of the inner shell (10).

2. Apparatus according to claim **1, characterised in that** the measuring element of said temperature sensor (36) is substantially halfway between the outer surface of the shaft (12) and the inner surface of the inner shell (10).

3. Apparatus according to claim 1 or 2, **characterised in that** said heating means extend from the first end (13) of the shaft (12) to the second end (15) of the shaft.

4. Apparatus according to any of claims 1-3, **characterised in that** there is at least one screw-thread-shaped blade (14) around the shaft (12) of the conveyor (3), which is attached by its first edge to the shaft (12) and whose free second edge extends close to the inner surface of the inner shell (10).

5. Apparatus according to claim 4, **characterised in that** the shaft (12) and the blade (14) are made of a material with good thermal conductivity, such as metal, preferably steel.

6. Apparatus according to claim 4 or 5, **characterised in that** the outer diameter of the first end (13) of said shaft (12) is larger than the outer diameter of the second end (15) of the shaft (12) and that the distance of the second edge of said blade (14) from the outer surface of the shaft (12) is smaller at the first end (13) of the shaft (12) than at the second end (15) of the shaft (12).

7. Apparatus according to any of claims 1-6, **characterised in that** said control unit (18) is configured to operate the conveyor (3) intermittently so that the conveyor (3) is operated when the temperature of the biomass is higher than the set target temperature and the conveyor (3) is stopped when the temperature of the biomass is lower than the set target temperature.

8. Apparatus according to any of claims 4-7, **characterised in that** said first temperature sensor (36) is attached to the inner surface of the inner shell (10) and that the second edge of said blade (14) has a recess at the location of said first temperature sensor (36).

9. Apparatus according to any of claims 1-8, **characterised in that** said shaft (12) has a tubular cavity (17) for the flow-through of a heat transfer fluid, which cavity (17) has a first end and a second end, and that said heating means comprise a heating boiler (20) for heating the heat transfer fluid, which heating boiler (20) is connected by a supply pipe (22) to the first end of the cavity (17) and by a return pipe (24) to the second end of the cavity (17), and a pump (26) for circulating the heat transfer fluid from the heating boiler (20) into the cavity (17) and back to the heating boiler (20).

10. Apparatus according to claim 9, **characterised in that** it comprises a second temperature sensor (38) connected to the control unit (18) for measuring the temperature of the heat transfer fluid returning from the shaft to the heating boiler (20).

11. Apparatus according to claim 9 or 10, **characterised in that** said heating means additionally comprise a flow channel (32) for the heat transfer fluid locating at least partially around the inner shell (10), which is connected directly or indirectly to said heating boiler (20).

12. Method for hygienising biomass, in which method
- a target temperature is set for the biomass to be hygienised,
- biomass to be hygienised is received into a tank (2),
- biomass is transferred out of the tank by a conveyor (3) having an initial end, an outlet end and an inner shell (3), and
- the biomass on the conveyor (3) is heated by heating means to the set target temperature or above at least partially by heating means inside the inner shell (10), **characterised in that** in the method the temperature of the biomass to be hygienised is measured at the initial end of the conveyor (3) by a first temperature sensor (36), the measuring element of which is extended into the biomass at a distance from the hottest surfaces of the conveyor.

13. Method according to claim 12, **characterised in that** in the method biomass is transferred by the conveyor (3) towards the outlet end only when the measured temperature exceeds the set target temperature.

14. Method according to claim 12 or 13, **characterised in that** a processing time is set for the biomass to be hygienised and the temperature of the biomass on the conveyor (3) is maintained at the target temperature or above for at least said processing time.

15. Method according to any of claims 12-14, **characterised in that** dry manure, bedded manure, biowaste, agricultural side stream material or naturally grown reed or straw material is hygienised by the method.
